# EUROPEAN PATENT SPECIFICATION

(11) **EP 1 494 705 B1**
(45) Date of publication and mention of the grant of the patent: **29.12.2010**
(21) Application number: 03745189.5
(22) Date of filing: 28.03.2003
(51) Int. Cl.: A61K 38/45, A61K 38/48, A61P 35/00, A61K 47/48

(54) **A COMPOSITION AND METHOD FOR KILLING OF TUMOURS**
ZUSAMMENSETZUNG UND VERFAHREN ZUR ABTÖTUNG VON TUMOREN
COMPOSITION ET PROCEDE DESTINES A TUER DES TUMEURS

(30) Priority: 28.03.2002 AU PR145602
(43) Date of publication of application: 12.01.2005
(73) Proprietor: COMMONWEALTH SCIENTIFIC AND INDUSTRIAL RESEARCH ORGANISATION, Campbell, ACT 2612 (AU)
(72) Inventor: BOTH, Gerald, Wayne, North Ryde, New South Wales 2113 (AU); LOCKETT, Trevor, John, Denistone, New South Wales 2114 (AU); MOLLOY, Peter, Laurence, Chatswood, New South Wales 2067 (AU); CAMERON, Fiona, Helen, Lindfield, New South Wales 2070 (AU); RUSSELL, Pamela, Joan, Drummoyne, New South Wales 2047 (AU); MARTINIELLO-WILKS, Rosetta, Mosman, New South Wales 2088 (AU); MOGHADDAM, Minoo, Jalali, Killara, New South Wales 2071 (AU); SMITH, Ian, Keith, Prospect, South Australia 5082 (AU)
(74) Representative: Chapman, Paul William
(86) International application number: PCT/AU2003/000381
(87) International publication number: WO 2003/082323

(56) References cited:
- WO-A1-00/52156
- WO-A1-96/03508
- AU-A- 2 786 800
- US-A- 5 854 224
- US-A- 5 906 922
- US-A- 5 906 922
- VOEKS, D. ET AL: "Gene therapy for prostate cancer delivered by ovine adenovirus and mediated by purine nucleoside phosphorylase and fludarabine in mouse models" GENE THERAPY , 9(12), 759-768 CODEN: GETHEC; ISSN: 0969-7128, 2002, XP002399217
- LOCKETT, LINDA J. ET AL: "Relative efficiency of tumor cell killing in vitro by two enzyme-prodrug systems delivered by identical adenovirus vectors" CLINICAL CANCER RESEARCH , 3(11), 2075-2080 CODEN: CCREF4; ISSN: 1078-0432, 1997, XP002399218
- MINCHEFF M ET AL: "Naked DNA and adenoviral immunizations for immunotherapy of prostate cancer: A phase I/II clinical trial" EUROPEAN UROLOGY, S. KARGER AG., BASEL, CH, vol. 38, no. 2, August 2000 (2000-08), pages 208-217, XP002957430 ISSN: 0302-2838
- FELGNER J H ET AL: "Enhanced gene delivery and mechanism studies with a novel series of cationic lipid formulations." THE JOURNAL OF BIOLOGICAL CHEMISTRY. 28 JAN 1994, vol. 269, no. 4, 28 January 1994 (1994-01-28), pages 2550-2561, XP002399219 ISSN: 0021-9258
- VOEKS D. ET AL.: 'Gene therapy for prostate cancer delivered by ovine adenovirus and mediated by purine nucleoside phosphorylase and fludarabine in mouse models' GENE THERAPY vol. 9, no. 12, June 2002, pages 759 - 768, XP002399217
- LU YI: 'Viral based gene therapy for prostate cancer' CURRENT GENE THERAPY vol. 1, 2001, pages 183 - 200, XP008073390
- SECRIST J. ET AL.: 'Gene therapy of cancer: activation of nucleoside prodrugs with E. coli purine nucleoside phosphorylase' NUCLEOSIDES & NUCLEOTIDES vol. 18, no. 4-5, 1999, pages 745 - 757, XP008073421
- ANDERSON L. ET AL.: 'Breast cancer specific expression of the candida albicans cytosine deaminase gene using a transcriptional targeting approach' CANCER GENE THERAPY vol. 7, no. 6, 2000, pages 845 - 852, XP008073389
- VRATI S ET AL: "Construction and transfection of ovine adenovirus genomic clones to rescue modified viruses" VIROLOGY 19960601 US LNKD- DOI:10.1006/VIRO.1996.0300, vol. 220, no. 1, 1 June 1996 (1996-06-01), pages 200-203, ISSN: 0042-6822
- CAMERON F H ET AL: "A TRANSFECTION COMPOUND SERIES BASED ON A VERSATILE TRIS LINKAGE" BIOCHIMICA ET BIOPHYSICA ACTA. BIOMEMBRANES, AMSTERDAM, NL LNKD- DOI:10.1016/S0005-2736(98)00248-X, vol. 1417, 1 January 1999 (1999-01-01), pages 37-50, XP001543164 ISSN: 0005-2736
- MARTINIELLO-WILKS R ET AL: "Gene-directed enzyme prodrug therapy for prostate cancer in a mouse model that imitates the development of human disease" JOURNAL OF GENE MEDICINE 200401 GB LNKD- DOI:10.1002/JGM.474, vol. 6, no. 1, January 2004 (2004-01), pages 43-54, ISSN: 1099-498X 1521-2254
- MARTINIELLO-WILKS R ET AL: "Purine nucleoside phosphorylase and fludarabine phosphate gene-directed enzyme prodrug therapy suppresses primary tumour growth and pseudo-metastases in a mouse model of prostate cancer" JOURNAL OF GENE MEDICINE 200412 GB LNKD- DOI:10.1002/JGM.629, vol. 6, no. 12, December 2004 (2004-12), pages 1343-1357, ISSN: 1099-498X 1521-2254
- WANG X Y ET AL: "Preclinical evaluation of a prostate-targeted gene-directed enzyme prodrug therapy delivered by ovine atadenovirus" GENE THERAPY 200411 GB LNKD- DOI:10.1038/SJ.GT.3302308, vol. 11, no. 21, November 2004 (2004-11), pages 1559-1567, ISSN: 0969-7128

## Description

### FIELD OF THE INVENTION

The present invention relates to a method of treating solid tumours involving the use of ovine atadenovirus gene transfer vectors expressing a suicide gene from a promoter and a prodrug that can effectively destroy tumours.

### BACKGROUND OF THE INVENTION

There is currently increasing interest in the use of gene directed enzyme prodrug therapy (GDEPT) in the treatment of cancer and there are several GDEPT systems are under investigation for cancer therapy. The most studied uses Herpes Simplex Virus-thymidine kinase (HSV-TK) gene transduction in combination with the prodrug ganciclovir (GCV). HSV-TK phosphorylates GCV, converting it to a nucleoside analogue that terminates DNA synthesis, leading to the death of dividing cells. A perceived advantage of the GDEPT system is its observed local "bystander effect", namely that more cells than are transduced die due to local spread of the toxic product. This occurs at least in part through intercellular transport of phosphorylated GCV via gap junctions.

One of the main difficulties encountered with GDEPT is the common issue encountered with other gene therapeutics that being one of delivery.

Numerous systems have been developed for the delivery of gene therapeutics. These range from retroviruses capable of stably introducing a gene of interest to the genome of recipient cells to non-viral systems such as cationic lipids and dendrimers. In particular, gene delivery systems based on adenoviruses are currently being used with increasing frequency. These viral vectors can be produced in high yield and transfer their genetic load to a broad range of target cell types with high efficiency. While human adenoviral vectors have attracted most attention, as endemic viruses in Man their utility as gene therapy vectors is seriously compromised by high levels of pre-existing immunity against them in the human population.

### SUMMARY OF THE INVENTION

The present inventors have developed a composition for use in GDEPT for solid tumours based on delivery using ovine atadenovirus.

Accordingly, in a first aspect, the present invention provides a composition for use in a method of treating a solid tumour in a subject, the method comprising the following steps
(i) delivering to the solid tumour a composition comprising an engineered ovine atadenovirus; and
(ii) administering a prodrug to the subject,
wherein the engineered ovine atadenovirus comprises a promoter and a gene encoding an enzyme which converts the prodrug to a cytotoxic metabolite, the gene being under the control of the promoter.

In a second aspect, the present invention provides a composition comprising
(i) an engineered ovine atadenovirus; and
(ii) a lipid,
wherein the engineered ovine atadenovirus comprises a promoter and a gene encoding an enzyme which converts a prodrug to a cytotoxic metabolite, the gene being under the control of the promoter.

### BRIEF DESCRIPTION OF FIGURES

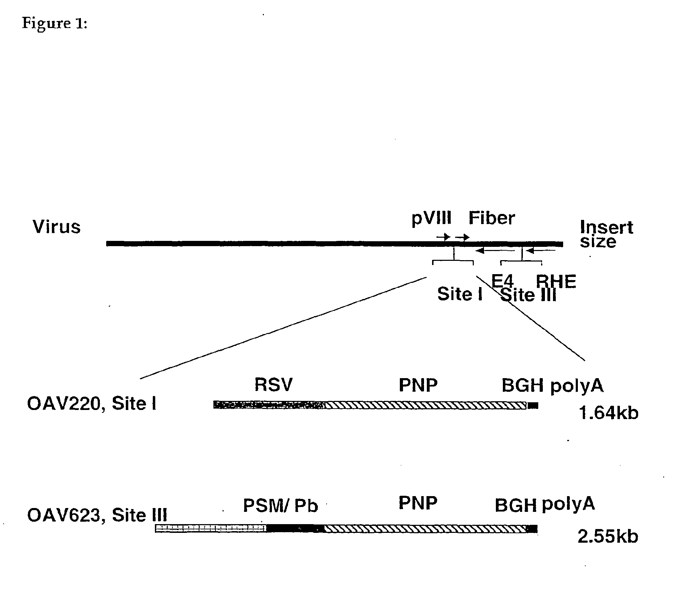

**Figure 1****.** Diagrammatic representation of recombinant ovine atadenoviruses used in the GDEPT studies described in the present specification. The diagrams also indicate the gene expression cassettes of the viruses. comparative OAdV220 expresses PNP from the constitutive RSV promoter (Long terminal repeat from the Rous Sarcoma Virus). The insertion is in site 1 in the OAdV genome. **OAdV222** is the same as OAdV 220 with the exception that the RSV promoter is replaced with the constitutive human cytomegalovirus immediate early promoter. **OAdV623** expresses PNP from the chimaeric PSM/Pb promoter. The PSM enhancer is depicted in vertical lines, the probasin promoter is solid. This promoter drives prostate-specific expression of PNP. This expression cassette is inserted into site III in the OAdV genome depicted at the top of the figure. **OAdV223** carries the same expression cassette as OAdV623 but inserted into site I of the OAdV genome. All expression cassettes carry the transcription termination sequences from the bovine growth hormone gene (BGH polyA).

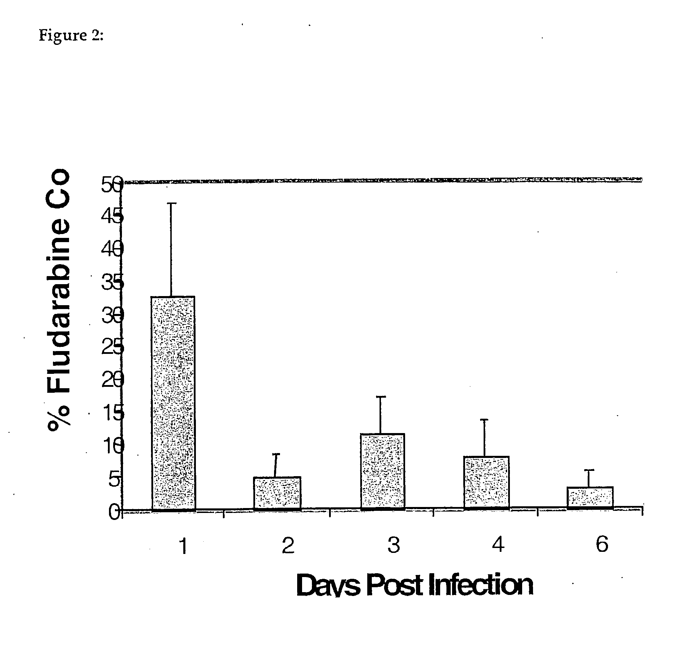

**Figure 2****.** In vitro conversion of Fludarabine to 2FA by PNP activity in RM1 tumour extracts. Tumours were inoculated with comparative OAdV220 at 6 x 10⁹ VP per tumour. Tumours were harvested 1-6 days after treatment and homogenised. Homogenates (500 µg of protein) were incubated with 500 n moles of Fludarabine for 16h at 37°C. % Conversion of Fludarabine to 2FA was determined by HPLC analysis.

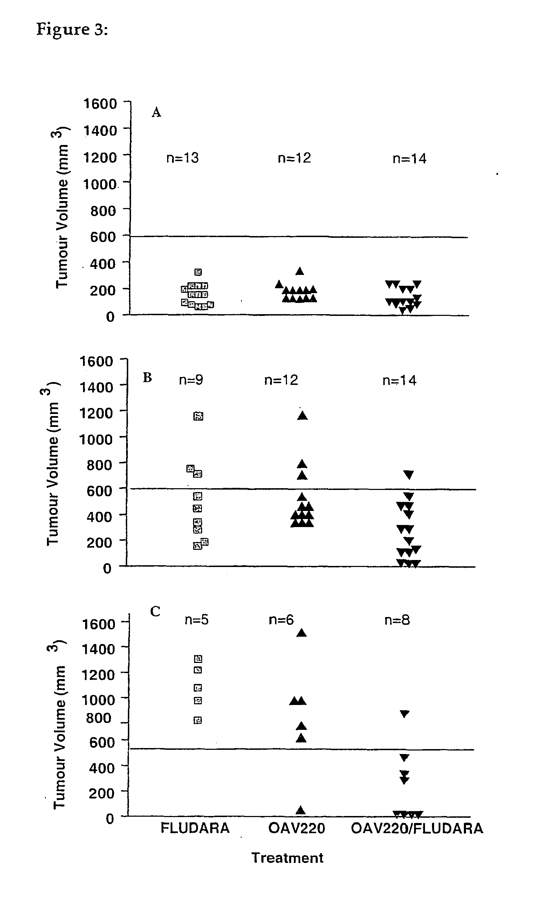

**Figure 3****.** Effect of OAdV and FLUDARA treatment on sc PC-3 tumour growth in nude mice. Animals were weighed, and tumour diameters were measured twice weekly. Data show the spread of tumour sizes and the number of survivors at days A, 4; B, 25 and C, 53 post-tumour injection for treatment and control groups. The line indicates half the maximum volume to which tumours were allowed to grow. Note that four tumours in the comparative OAdV220/Fludarabine treatment group had completely regressed by day 53.

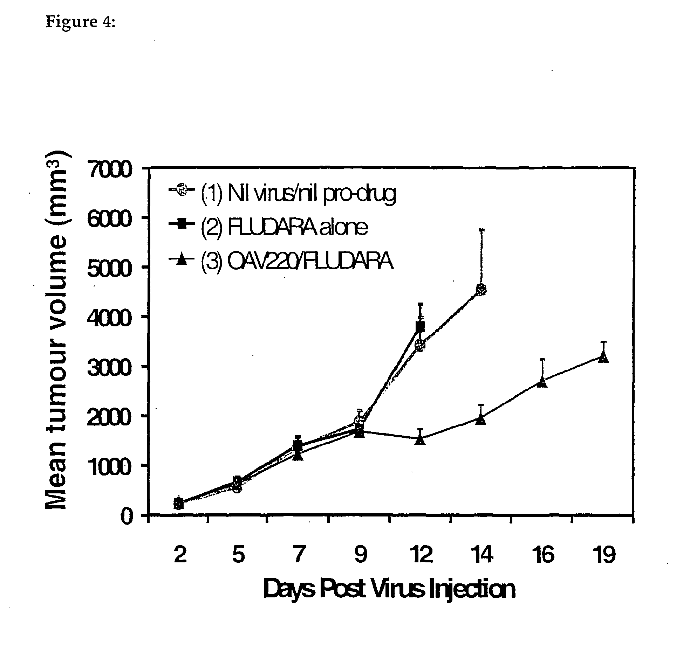

**Figure 4****.** Effect of fludarabine and comparative OAdV220 transduction on the growth of RM-1 tumours.

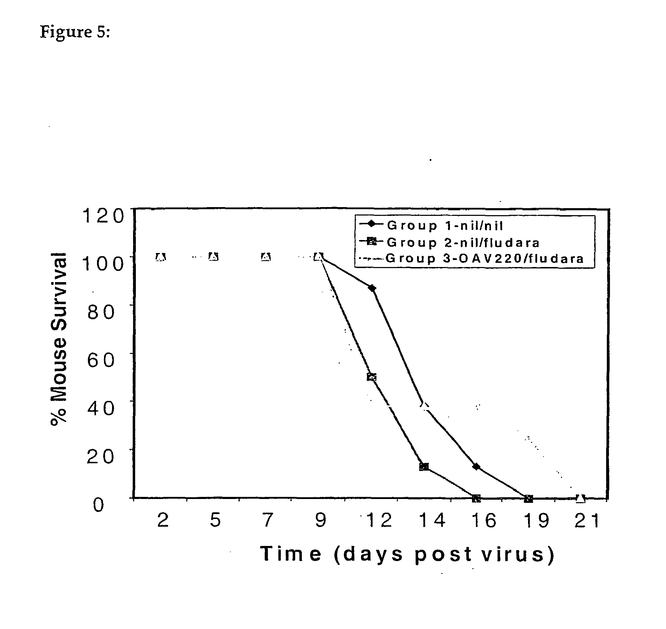

**Figure 5****.** Effect of fludarabine and comparative OAdV 220 on survival of animals bearing RM-1 subcutaneous tumours.

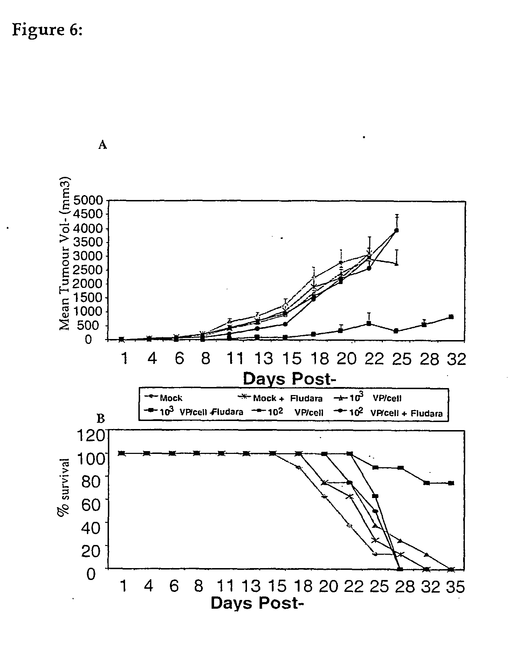

**Figure 6****.** Effect of pre-transduction of RM-1 cells with comparative OAdV220 on GDEPT in subcutaneous tumours. RM-1 cells were pre-treated with comparative OAdV220 or virus storage buffer as indicated in the key on day -1. Mice received daily intraperitoneal (ip) injections of either fludarabine or saline. A, Impact of GDEPT on tumour volume (mean + SE). B, Impact of GDEPT on mouse survival.

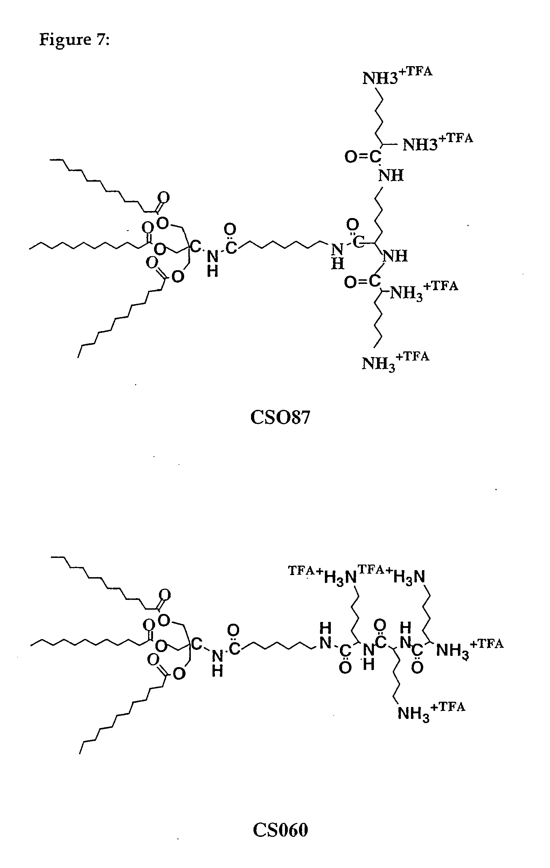

**Figure 7****.** Examples of Tris conjugate cationic lipids.

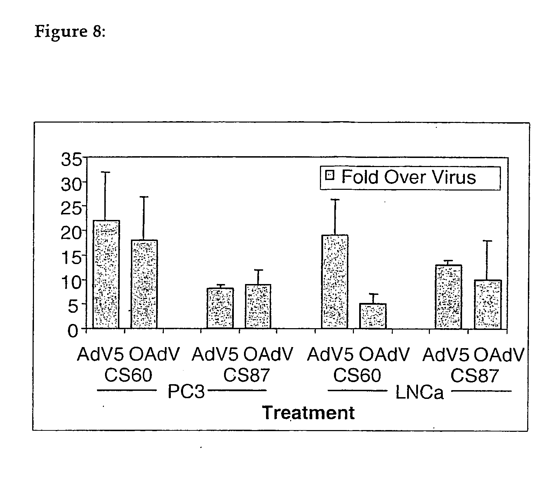

**Figure 8****.** Effect of Tris-conjugated cationic lipids on vector transduction in prostate cancer cells in vitro. The AdV5 and OAdV vectors carried reporter gene cassettes encoding *E. coli-*galactosidase and human placental alkaline phosphatase, respectively. Levels of expression obtained in the presence of the lipids indicated (CS60 and CS87) are expressed relative to those achieved with the same virus in the absence of lipid. Transduction experiments were performed *in vitro* in two human prostate cancer cell lines, PC3 and LNCaP. Bars indicate Standard Errors.

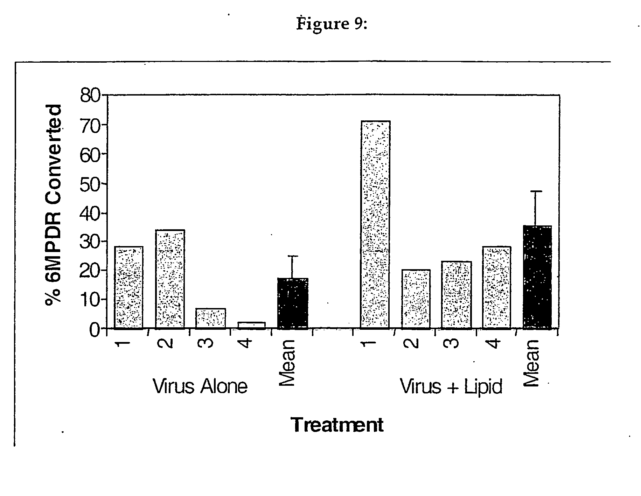

**Figure 9****.** Lipid enhancement of OAdV transduction *in vivo.* Human LN3 prostate cancer tumours were grown subcutaneously in nude mice. Tumours were injected with 10¹⁰ VP of OAdV623 (a vector carrying the *E coli* purine nucleoside phosphorylase (PNP gene) under the control of the PSM/Pb promoter) in the presence or absence of cationic lipid CS87. Tumours were excised 3 days later, homogenised and efficiency of transduction was determined by an assay for PNP activity. Tumour lysate protein (500µg) was incubated with the PNP substrate 6MPDR for 2h at 37°C and the amount of conversion to 6MP was determined by HPLC analysis. Absolute values for each tumour plus mean values and standard errors for the different treatments are shown.

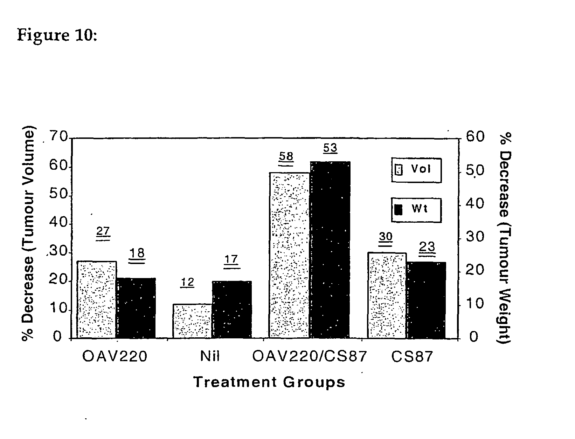

**Figure 10**. Effect of GDEPT ± lipid on growth of intraprostatic RM-1 tumours. RM-1 prostate tumours were established orthotopically and treated as indicated on the X-axis. Tumours were removed on day 18 and their volumes and weights determined. Data represent the reduction in tumour volume or weight observed for any given treatment in the presence of Fludarabine relative to that of the equivalent non-Fludarabine treated tumour expressed as a % of the volume or weight of the non-prodrug treated tumour.

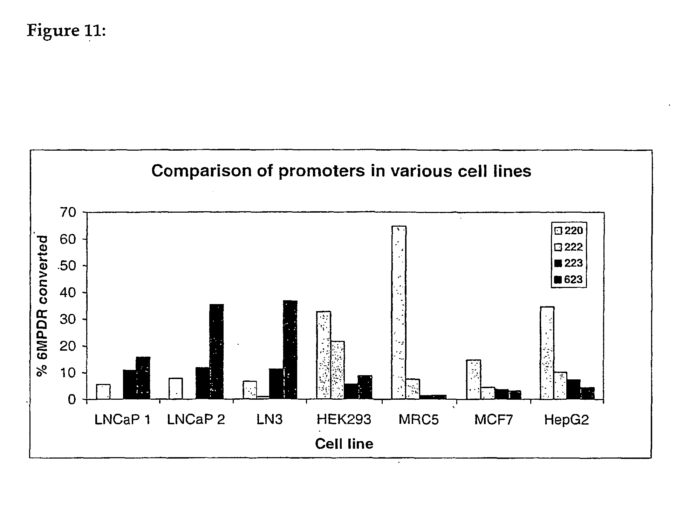

**Figure 11****.** Comparison of PSM/Pb and non-specific promoter activities during OAdV infections in various cell types. PNP production was measured by 6MPDR conversion. As not all cell types were equally infected by OAdV and different amounts of cell lysate were assayed, only the ratio of activities within a cell type should be compared. These human cell lines were derived from breast (MCF7) or liver (HepG2) or prostate (LNCaP, LN3) cancers. MRC5 cells are normal human lung fibroblasts.

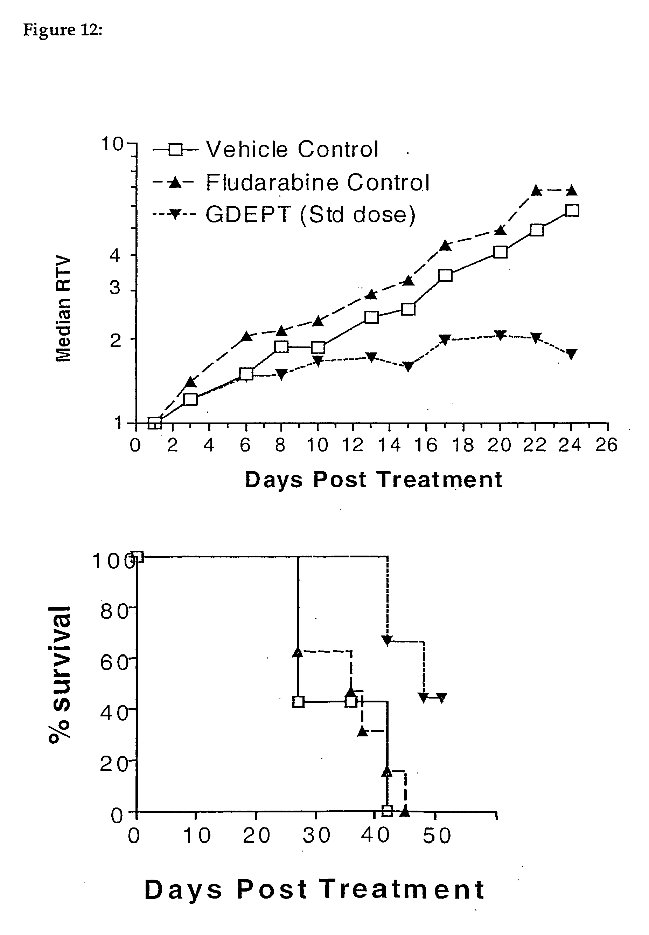

**Figure 12****.** Effect of prostate specific expression of the GDEPT on growth of human prostate tumours in vivo and on host survival (nude mice). Tumours were grown subcutaneously in nude mice from human LN3 prostate cancer cells. Tumours were injected once with vehicle only (Vehicle Control and Fludarabine Control) or OAdV623 at 10¹⁰ VP/tumour plus 10 µM CS87 (GDEPT). Fludarabine Control and GDEPT groups received fludarabine for 5 days post virus injection. Treatment groups are indicated in the key. Upper; effect on tumour growth plotted as median relative tumour volume (RTV). Lower; effect on mouse survival.

### DETAILED DESCRIPTION

In a first aspect, the present invention provides a composition for use in a method of treating a solid tumour in a subject, the method comprising the following steps
(i) delivering to the solid tumour a composition comprising an engineered ovine atadenovirus according to claim 1; and
(ii) administering a purine prodrug to the subject,
wherein the engineered ovine atadenovirus comprises a promoter and a gene encoding an enzyme which converts the produg to a cytotoxic metabolite, the gene being under the control of the promoter. It is preferred that the solid tumour is prostate cancer.

The promoter is the probasin promoter. The ovine atadenovirus further comprises a transcriptional enhancer element, from the prostate specific membrane antigen gene.

The enzyme is a purine nucleoside phosphorylase (PNP), and the prodrug is a purine prodrug which is converted by PNP to a toxic purine metabolite. The currently preferred prodrugs are 6-methyl purine-2-deoxyriboside (6MPDR) and fludarabine; the toxic products produced by PNP on these substrates are 6-methyl purine (6MP) and 2-fluoroadenine (2FA), respectively.

Fludarabine is approved for clinical use but its Km for PNP is a ∼1000-higher than that for 6MPDR. However, its PNP metabolite, 2FA is ∼100-fold more potent as a growth inhibitor than 6MP, the metabolite of 6MPDR. Through their incorporation into RNA as well as DNA the prodrug as well as the metabolites produced by this enzyme/ prodrug system together kill non-dividing as well as dividing cells.

This enzyme/ prodrug system also induces a more efficient local "bystander effect" than the HSV-TK/GCV system because the metabolites are non-phosphorylated purines that can diffuse freely across the membranes of non-transduced cells.

The ovine atadenovirus (OAdV, formerly designated OAV) is preferably the ovine adenovirus described in PCT/AU95/00453 and/or PCT/AU96/00518. The name of the virus has been changed to reflect its recognition in March 2002 by the International Committee for the Nomenclature of Viruses as the prototype of the new atadenovirus genus. It is thus distinct from other ovine adenoviruses that are in the mastadenovirus genus. The sequence of OAdV is provided in GenBank Accession No. U40839.

The cloning of a full length, infectious OAdV287 genome into the plasmid vector pBluescribe has been described (Vrati S, Macavoy ES, Xu ZZ, Smole C, Boyle DB and Both GW (1996) "Construction and transfection of ovine atadenovirus genomic clones to rescue modified viruses", Virology 220: 200-203). This primary recombinant product, pOAdV100, allows for the convenient release of the linearised, infectious OAdV287 genome by digestion with the restriction endonuclaese KpnI. Further modifications to pOAdV100 have been made to allow convenient insertion of foreign DNA into the viral genome without disrupting normal virus replication and packaging functions. One such modified plasmid is pOAdV600. To create pOAdV600 the ∼7.1 kb SphI/SaII fragment of pOAdV100 was subcloned into pALTER-1(Promega Corp., Madison, WI). Using a mutagenesis kit (Altered sitesII, Promega Corp.), unique ApaI and NotI sites were inserted using a synthetic oligonucleotide (5' ......GGG CCC AGA TAT CAG CGG CCGC ......3' ) where the flanking sequences (indicated by the dots) were designed to allowed insertion of the oligonucleotide sequence indicated immediately 5' to base 26,676 of the OAdV287 sequence. The modified SphI/SaII fragment was then recloned into SphI/SaII cut pOAdV100 to produce pOAdV600. In a related fashion, the same sequence indicated above was inserted between nucleotides 22129 and 22130 of the OAdV287 genomic sequence resulting in the production of pOAdV200. Both pOAdV200 and pOAdV600 have been used to prepare recombinant ovine atadenoviruses carrying a variety of gene expression cassettes including cassettes useful for a PNP-based GDEPT for the treatment of cancers. The insertion points in these plasmids are referred to as sites I and III, respectively.

By varying the promoter used to drive expression of the enzyme component of a GDEPT, viruses can be tailored either for utility in the treatment of a wide range of tumours or to provide targeted tumour type and/or tissue specificity.

comparative OAdV220 provides an example of a therapeutic virus that may be used in the treatment of a wide variety of tumours. In this virus transcription of the PNP gene from E. coli is driven by the constitutive RSV promoter (3' long terminal repeat from the Rous Sarcoma Virus) and terminated by the polyadenylation sequence from the bovine growth hormone gene. This expression cassette is inserted into ApaI/NotI polylinkerof pOAdV200 (ie is in site 1 of the OAdV287 genome). Use of a strong constitutive promoter such as that found in the RSV 3' long terminal repeat allows for high levels of PNP expression in a wide variety of cell types and tissues.

On the other hand, to maximise the local effect of GDEPT while minimising the potential for non-specific toxicity (eg. through virus uptake in the liver or other tissues surrounding the injection site) it is desirable to restrict expression of the prodrug activating enzyme to target tissues. For any particular cancer type this can be achieved by placing the GDEPT gene expression cassette under the control of a promoter that is active only in the tissue type from which the cancer is derived. In the case of prostate cancer a candidate for such a gene regulator is the promoter for prostate specific membrane antigen.

Prostate specific membrane antigen (PSMA) is a protein with folate hydrolase activity. It was first identified as an antigen present on the membrane of a prostate cancer cell line as well as normal and cancerous prostate epithelium. Its expression is largely restricted to the prostate. Lower levels of expression have been detected in the brain, the small intestine and in a subset of kidney tubule cells (reviewed in (Fair et al., 1997. Prostate-specific membrane antigen. Prostate 32(2), 140-148). More recently it was established that PSMA is also expressed in the neovasculature of a wide range of tumour types, but not in normal vasculature (Chang et al., 1999, Five different anti-prostate-specific membrane antigen (PSMA) antibodies confirm PSMA expression in tumour-associated neovasculature. Cancer Res 59(13), 3192-3198; Liu et al. 199,. Monoclonal antibodies to the extracellular domain of prostate-specific membrane antigen also react with tumour vascular endothelium. Cancer Res 57(17), 3629-3634; Silver et al., 1997, Prostate-specific membrane antigen expression in normal and malignant human tissues. Clin Cancer Res 3, 81-85). Both cDNA and genomic clones of PSMA have been isolated and characterised (Israeli et al., 1993, Molecular cloning of a complementary DNA encoding a prostate-specific membrane antigen. Cancer Res 53, 227-230; O'Keefe et al., 1998, Mapping, genomic organization and promoter analysis of the human prostate-specific membrane antigen gene. Biochimica Et Biophysica Acta 1443: 113-27).

A transcriptional enhancer sequence (PSME) has been isolated from the PSMA gene and demonstrated in a series of transfection studies in multiple cell types to confer high level enhancement of transcription that is restricted to PSMA-expressing prostate cells. PSME has been shown to activate transcription from both its own promoter and those of heterologous genes. Further information regarding this enhancer element may be found in PCT/AU00/01143.

The 430 bp proximal promoter from the rat probasin gene has also been shown to direct expression specifically to prostate cells both *in vitro* and in transgenic mice (Brookes et al., 1998, Relative activity and specificity of promoters from prostate-expressed genes. Prostate 35(1),18-26; Greenberg et al., 1994, The rat probasin gene promoter directs hormonally and developmentally regulated expression of a heterologous gene specifically to the prostate in transgenic mice. Mol Endocrinol 8(2), 230-239; Kasper et al., 1994, Cooperative binding of androgen receptors to two DNA sequences is required for androgen induction of the probasin gene. J Biol Chem 269(50), 31763-9). A chimaeric promoter consisting of PSME and the probasin proximal promoter, when linked to a reporter gene, produced expression levels of the reporter product that were about ten times higher than those obtained with PSME linked to its own, PSMA, promoter. Importantly the tissue specificity of expression was also maintained.

In OAdV623, transcription of the PNP gene is under the control of the chimaeric promoter described above, ie. an ∼ 1kb fragment including bases 14760 - 15804 of the human prostate specific membrane antigen gene sequence (O'Keefe et al., 1998, Mapping, genomic organisation and promoter analysis of the human prostate-specific membrane antigen gene. Biochimica Et Biophysica Acta 1443(1-2), 113-27) operatively coupled to the 430 bp proximal promoter from the rat probasin gene. Transcription of this expression cassette is terminated with the bovine growth hormone poly adenylation sequence (BGH Poly A). This expression cassette is inserted into site III of the OAdV287 sequence. OAdV623, therefore, provides an example of a therapeutic virus that can find greatest utility in the delivery of a GDEPT for the treatment of prostate cancer.

The composition comprising the engineered ovine atadenovirus further comprises a lipid. The lipid is a cationic lipid. Cationic lipids based on a TRIS linkage are described in US patents 5,854,224 and 5,906,922. The cationic lipids are shown in Figure 7.

The lipid is CSO87 having the formula : 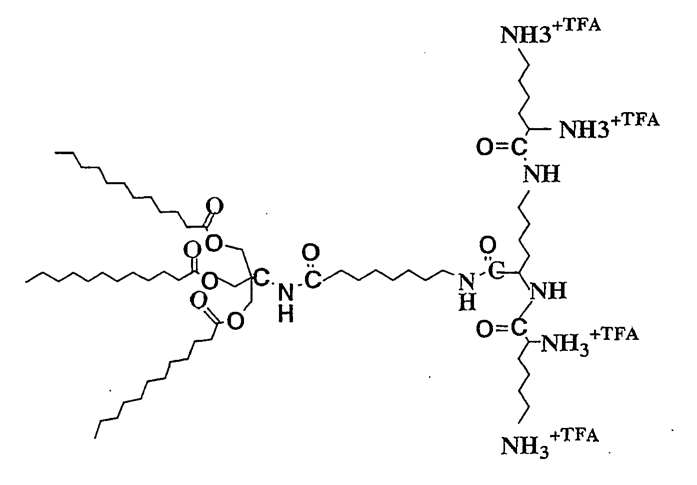 or CSO60 having the formula:

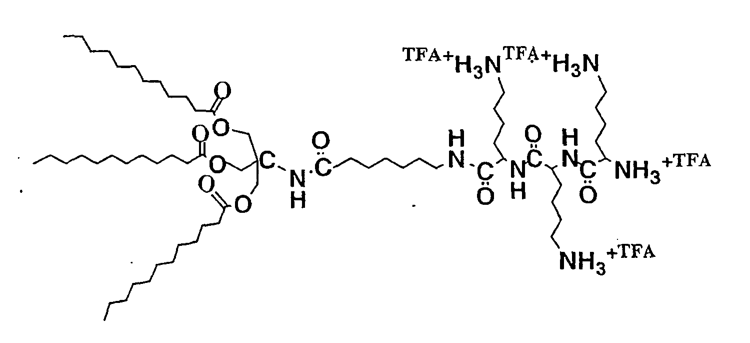

The composition of the present invention preferably comprises an engineered ovine atadenovirus selected from the group consisting of OAdV223 and OAdV623.

It is preferred that the claimed composition comprising the engineered ovine atadenovirus and cationic lipid is used to directly deliver the composition to the solid tumor by injecting into the tumor.

As used herein the term "treating" is used in its broadest sense and is intended to encompass treatment that results in eradication of the tumour, causes a decrease in tumour size or causes a decrease in rate of tumour growth.

Cationic lipids have been shown to facilitate transfer of DNA into cells and to enhance the infectivity of a range of viruses. In addition, lipids are also known to enhance immune responses when complexed with specific antigens. Lipids and lipopeptides, when formulated with specific peptides or antigenic agents have been shown to potentiate the immune responses to these agents. Vaccination of this sort leads to production of both Th1 and Th2 immune responses enhancing the immune response raised against weakly immunogenic antigens.

It has also been shown that immune responses against specific tumours can be elicited by vaccination of tumour-bearing animals with antigens specific for that tumour (often prepared by recombinant DNA approaches) formulated with cationic lipids. Such vaccination suppressed both primary and metastatic tumour growth.

In summary, the present inventors have shown that cationic lipids can also enhance the ability of ovine atadenovirus gene transfer vectors to infect a range of cell types including prostate cancer cells. The inventors have developed ovine atadenovirus vectors that carry the *E*. *coli* gene encoding purine nucleoside phosphorylase operably linked to either the highly active, constitutive promoter of the Rous sarcoma virus 3' long terminal repeat for comparison or the prostate-specific, chimaeric PSM enhancer/probasin promoter and the bovine growth hormone poly adenylation sequence. Schematic representations of these recombinant viral genomes are shown in Figure 1. The inventors have demonstrated that injection of these viruses complexed with said cationic lipids into tumours carried in mice and derived from prostate cancer cells coupled with the administered prodrug fludarabine leads to a significant reduction in tumour growth over that observed with virus alone.

### Example 1. OAdV Delivers Transgenes Genes into Tumours in vivo.

To determine whether OAdV could deliver genes into tumours *in vivo,* cells from the human prostate cancer, androgen independent cell line PC3 (1.5 x 10⁶ cells/tumour) were implanted subcutaneously (sc) in BALB/ c (nu/nu) ["nude"] mice. Tumours were allowed to develop for 3-6 weeks until they had grown to around 5 mm in diameter. Tumours were then injected with 1.2x10⁸ plaque forming units (pfu) of OAdV216, an ovine atadenoviral vector expressing the human placental alkaline phosphatase gene. Tumours were harvested four days post injection, frozen, sectioned and stained for reporter gene activity.Extensive alkaline phosphatase staining of a representative tumour section was observed indicating that wide dissemination of the virus within the tumour and infection of tumour cells occurred.

### Example 2. OAdV Delivers PNP Activity into Tumours in vivo.

RM-1 is an androgen-independent cell line derived by transformation of cells from the genital ridge of embryonic C57BL/ 6 mice with *ras* and *myc* oncogenes. When implanted into C57BL/6 mice in the appropriate locations these cells can form tumours either subcutaneously or in the prostate. Lung pseudometastases can also be formed when cells are injected intravenously (iv) via the tail vein (Hall et al., 1997, Adenovirus-mediated herpes simplex virus thymidine kinase gene and ganciclovir therapy leads to systemic activity against spontaneous and induced metastasis-in an orthotopic mouse model of prostate cancer. Int J Cancer70, 183-187; Hall et al.1998, Induction of potent antitumor natural killer cell activity by herpes simplex virus thymidine kinase and ganciclovir therapy in an orthotopic mouse model of prostate cancer. Cancer Res 58: 3221-3225).

To determine whether, in an immunocompetent animal, a single intratumoural administration of a PNP-expressing OAdV virus could lead to PNP expression that would be sustained over a period of prodrug treatment that might be used for therapy, subcutaneous RM-1 tumours were established in C57BL/6 mice. Tumours were initiated by injection of 2.5 x 10⁵ RM-1 cells into the flanks of C57BL/6 mice. On day 0 when tumours had reached 5 mm diameter they were injected with 20 µl of a preparation of OAdV220 (Figure 1) containing 6 x 10⁹ virus particles (VP) of virus in virus storage buffer. Tumours were excised,1-6 days after treatment and homogenised in 400 µlof ice cold homogenisation buffer (50 mM potassium phosphate buffer, pH 7.4). Homogenates were transferred to clean eppendorf tubes, snap frozen on dry ice then thawed in a 37°C water bath. Tubes were mixed again and subjected to two further rounds of freeze thaw treatment to complete cell lysis. Cell debris was removed by centrifugation for 5 minutes at 15,000 x g. Supernatants were removed to new sterile microfuge tubes and placed on ice. Aliquots from each sample were removed for estimation of protein content using a Pierce BCA protein estimation kit. Volumes of homogenate containing 500 µg of soluble protein were removed to fresh tubes for quantification of PNP activity.

To assay for PNP activity, phosphate buffer (described above) was added to the homogenate to a final volume of 1.1 mL. To each sample was added 100 µl of phosphate buffer containing 500 nmols of fludarabine (substrate for the PNP assay). Contents were gently mixed then tubes were incubated at 37°C for 16h. The reaction was stopped by heating the samples to 100°C for 5 min. Tubes were then centrifuged at 15,000 x g for 5 min to remove any remaining debris. The amount of fludarabine converted to 2FA by the PNP present in the tumour lysates was determined by analytical high performance liquid chromatography.

For substrate/product separation a Millipore C18 column was equilibrated with 50 mM NH₄H₂PO₄/5% Acetonitrile for 30 min (flow rate 1 mL/min). Sample (50µl) was applied to the column and fractionated in the same ammonium phosphate/ acetonitrile buffer (isocratic gradient) at a flow rate of 1.5 ml/min. The elution profile was detected by UV absorbance at 254 nm and the relative amounts of material in the substrate and product peaks were determined by calculating the area under the curve for each peak. These HPLC analyses showed that, under these conditions, the different lysates converted between 5 and 35% of available substrate to 2FA (Figure 2). The results confirm that OAdV220 can effectively deliver the PNP gene expression cassette into tumours *in vivo* in an immunocompetent host and that PNP expression can be sustained over at least a six day period.

### Example 3. OAdV Delivers Genes to Primary Human Prostate Tissue.

As a bridge between our studies in the animal models and clinical studies we have examined whether our viral vectors can deliver reporter genes to post-operative human prostate tissues. Tissue slices, obtained from either transurethral resections of the prostate (TURP) or from radical prostatectomy specimens from patients undergoing surgery for various conditions of the prostate, were cut into small fragments. These fragments of prostate tissue were placed on matrigel on pieces of gel foam in tissue culture wells and culture medium (T-medium (Thalmann GN, Sikes RA, Chang F-M, Johnston DA, von Eschenbach AC and Chung LWK "Suramin-introduced decrease in prostate specific antigen expression with no effect on tumour growth in the LNCaP model of human prostate cancer" J. Nat. Cancer Inst. 88: 794-801) was placed in the wells to the level of the gel foam, but not covering the tissue. After overnight incubation the tissues were transduced with OAdV217A, a virus carrying the Green Fluorescent Protein (GFP) reporter gene under the control of the cytomegalovirus (CMV) immediate early promoter. Green fluorescence of the tissue provided evidence for virus transduction. Viability of the tissue was assessed by propidium iodide exclusion. While this is not a quantitative assay, the results obtaineddemonstrated that, where the tissue was viable, the OAdV vector successfully transduced the primary human prostatic tissue. Samples successfully transduced ranged from benign hyperplastic to high-grade tumour tissue.

### Example 4. Ovine atadenovirus-borne, PNP-based GDEPT suppresses growth of human prostate cancers in nude mice.

The ability of the PNP-based GDEPT system to suppress tumour growth *in vivo* was tested using subcutaneous human PC3 tumours grown in nude mice. Once the tumours were established (see example 1), on day 0 two groups of mice received one intratumoural injection of 1 x 10¹⁰ VP of OAdV220. Two further groups received virus storage buffer only. Following this treatment all animals in one of the virus groups and all animals in one of the no-virus groups received a daily intraperitoneal (ip) injection of fludarabine (75 mg/ m²/ day) for the next five days. Animal weights and tumour volumes were recorded twice weekly. The vector only group was compared to the nil virus, nil prodrug group. The group that received vector plus fludarabine was compared with the group that received fludarabine only. The OAdV220 vector alone caused a 30% reduction in tumour growth. However, the overall tumour growth suppression was increased to 71% when virus was used in conjunction with Fludarabine.

Figure 3 shows the data presented as the number of responding tumours at day 4 (just after the start of treatment), day 25 (whilst control mice are still alive) and day 53, after all control mice had died. The cut off for response was arbitrarily taken as a tumour volume of 600 mm³, half the maximum tumour volume (1200 mm³) allowed before mice had to be culled because of tumour burden. By day 25, 13 of 14 tumours treated with OAdV220 plus fludarabine were in the responder category compared with 6 of 9 that received fludarabine only, and 9 of 12 which received virus only. By day 53, corresponding figures were 7/8 responders (OAdV plus Fludarabine), 1/6 (OAdV220 only) and 0/5 (Fludarabine only). By day 53 post treatment, 57% of mice receiving OAdV220 plus Fludarabine were alive compared with 14% receiving vector alone.

### Example 5. Ovine atadenovirus-borne, PNP-based GDEPT suppresses growth of murine tumours in immunocompetent mice.

To confirm that the GDEPT treatment could suppress tumour growth in the presence of a functioning immune system, C57BL/6 mice were inoculated subcutaneously with 2.5 x 10⁵ RM-1 tumour cells on day -5 to establish tumour growth. On day 0, these tumours were injected with OAdV220 at 6 x 10⁹ VP/tumour. On days 1-5 mice received either 600 mg/m²/day of Fludarabine or an equivalent volume of saline by intraperitoneal administration. Animal weights and tumour volumes were recorded twice weekly. Figure 4 shows that OAdV plus Fludarabine reduced overall tumour growth by 35% when compared to controls. At day 12, when group comparison was still possible (i.e., control mice were still alive), there was a 59% reduction in mean tumour volume in the OAdV220/fludarabine group compared with fludarabine alone. Figure 5 shows that 30% of OAdV220/Fludarabine treated mice survived for an extra 3 days compared with control mice which all died by day 16. These.results are particularly encouraging given the aggressiveness of this particular tumour model.

### Example 6. Increasing transduction levels of RM-1 tumour cells enhances the antitumour effect of an OAdV-borne GEDEPT on subcutaneous prostate tumours in immunocompetent mice.

RM-1 tumours provide a very aggressive cancer model. Thus the effects shown in example 5 are likely to under estimate the efficacy that might be expected in the treatment of human disease. It was thought that a better model for treatment of human disease, where prostate cancers grow more slowly, might be achieved if the tumours could be transduced with virus earlier than 5 days post inoculation. In practice, however, it is too difficult to accurately inject tumours earlier than day 5 in this model. An alternative approach was therefore adopted in which RM-1 tumour cells were transduced with virus *in vitro* prior to their inoculation into mice.

RM-1 cells (3 x 10⁷) were transduced in culture with 1 x 10³ or 1 x 10² VP/cell of OAdV220 or mock transduced with virus dilution buffer on day -1. On day 0,12 mice/group were subcutaneously injected with 2.5 x 10⁵ virus- or mock-transduced cells. Fludarabine was then administered from days 1-5 at 600 mg/m²/day. Tumour volume and animal survival were monitored three times per week. A line of best fit was generated for each treatment curve and the ability to suppress tumour growth or increase animal survival was determined from the days taken to reach the midpoint of tumour growth or animal survival respectively.

Figure 6A traces the increase in mean tumour volume with time after implantation. The GDEPT effect showed a clear dose response related to the virus input/cell. At day 15 post treatment, cells transduced with 1 x 10³ VP / cell plus Fludarabine showed a 90% reduction in mean tumour volume compared with mock transduced cells that also received Fludarabine. In contrast cells transduced with 1 x 10² particles/cell and receiving Fludarabine had only a 41% reduction in mean tumour volume compared with Fludarabine treated, mock transduced cells. Fludarabine alone caused a 17% reduction in mean tumour volume compared with nil treatment at day 15. Overall, when cells were pre-transduced with 1 x 10³ VP/ cell and receptor animals were treated with Fludarabine, a 74% reduction in tumour growth was observed when compared with tumours derived from mock-transduced cells where the host animals also received Fludarabine. Figure 6B shows that survival was markedly increased by GDEPT treatment. 80% of mice receiving cells transduced with 1 x 10³ VP / cell in combination with the prodrug were still alive at day 34 whereas all control mice had died by day 28. There was no improved survival of animals receiving RM-1 cells treated with 1 x 10² VP/cell plus Fludarabine compared with the matched minus Fludarabine control. These results suggest a dose responsiveness to the viral input, which presumably reflects a dose responsiveness to the amount of PNP product.

### Example 7. Cationic lipids enhance virus transduction of prostate cancer cells both in vitro and in vivo.

To investigate whether cationic lipids could be used to enhance viral transduction of human prostate cancer cells, two human prostate cancer cell lines, PC3 and LNCaP, were cultured in 96 well plates and treated with either a human mastadenovirus (AdV5) or an ovine atadenovirus carrying a reporter gene, ± cationic lipid and the relative amounts of reporter gene expression were determined. PC3 cells were cultured in RPMI medium + 10% FCS. LNCaP cells were cultured as previously described (Thalmann GN et al 1996 "Suramin-induced decrease in prostate-specific antigen expression with no effect on tumour growth in the LNCaP model of human prostate cancer". J Nat. Cancer Inst 88: 794-801). On day -1 cells were split into the wells of a 96 well culture tray (2 x 10⁴ cells per well for both cell lines; for LNCaP cells, wells of the culture trays were pre-treated with 10% fibronectin for 30 min prior to addition of cells to enhance the adherence of the cells to the tray during cell culture and the various washing steps involved in the enzymatic assays). On day 0 four identical serial dilutions of virus in serum free medium were prepared across the columns of a microtitre plate (50, 25, 12.5 and 6.25 x 10⁸ VP per 100 µl). In another microtitre tray four identical serial dilutions of Tris-conjugated cationic lipid (CS60 or CS87 see Figure 7), were prepared down the rows of the plate (21, 10.5, 5.25 and 0 µM, volume 100 µl). Virus dilutions (100 µl) were transferred to the corresponding wells in the lipid tray. The mixture was allowed to stand at room temperature for 15 min before application to cells. Cultures were returned to the CO₂ incubator for four hours to allow viral transduction. Foetal Calf Serum was then added to a final concentration of 10% and the plates were returned to the incubator. Two days after viral infection the viability of the cultures was examined using the MTS assay. Wells were then washed twice with PBS, cell lysis buffer (50 µl, 10mM Tris, 0.2% Triton X100, pH 8.0) was added to each well and plates were frozen at -70°C.

The expression cassette in the human AdV5 vector contained the *E coli* -beta galactosidase gene under the control of the RSV promoter. Assay for transgene expression was essentially as described in Felgner JH et al. 1994, (Enhanced gene delivery and mechanism studies with a novel series of cationic lipid formulations. J Biol Chem 269:2550-61). 100 µl of substrate buffer (1 mg/ml chlorophenol red, galactopyranoside in 60 mM sodium phosphate buffer pH 8.0, 1 mM magnesium sulphate, 10 mM potassium chloride, 50 mM beta-mercaptoethanol) was added to each well and colour was allowed to develop at room temperature. Beta-galactosidase activity was quantified relative to a standard curve prepared from purified enzyme

The expression cassette in the OAdV vector carried the human placental alkaline phosphatase reporter gene. Transduction, viability assays and cell lysis steps were all performed as described above. Upon thawing, the wells were covered with parafilm and plates incubated for 30 min at 65°C. Samples were cooled on ice then 25 µl of each lysate was transferred the wells of a clean, black microtitre plate. Human placental alkaline phosphatase activity was quantified using the Roche Molecular Biochemicals "AttoPhos substrate set", Cat# 1681 982 by reading fluorescence from each sample against those obtained from a standard curve prepared from purified enzyme.

Formulation of both AdV5 and OAdV with Tris-conjugated cationic lipids significantly enhanced viral transduction of human prostate cancer cells in culture. Typically enhancements of around 15 fold and 9-18 fold are observed for the AdV5 and OAdV vectors respectively (Figure 8).

Lipid also enhanced viral transduction of tumours growing *in vivo.* Tumours derived from the human prostate cancer cell line LN3 were established in male nude mice by subcutaneous injection of 2 x 10⁶ cells diluted 1:1 with matrigel (100µl) and allowed to grow to a size of 5 x 5 mm. Tumours were injected with 10¹⁰ VP of OAdV623 (a vector carrying the *E. coli* purine nucleoside phosphorylase (PNP gene) under the control of the PSM/Pb promoter (Figure 1) in the presence or absence of cationic lipid CS87 (4 tumours per group). Tumours were excised 3 days later and homogenised as described in Example 2. Samples of homogenates containing 500 µg of soluble protein (1.1 mL) were incubated with 100 µl of 5 mM 6MPDR for 2 h at 37°C. The amount of 6MPDR converted to 6MP by the PNP present in the tumour lysates was determined by analytical high performance liquid chromatography under conditions identical to those described in Example 2. Figure 9 shows absolute percentage conversion values calculated for each tumour plus mean values and associated standard errors for all tumours in the different treatments. These experiments revealed that, at the viral input tested both the number of injected tumours expressing PNP and the mean level of transgene expression was higher in tumours treated with the lipid / virus formulation.

### Example 8. Effect of GDEPT ± lipid on intraprostatic RM-1 tumours.

In order to mimic clinical prostate cancer in an immunocompetent animal model, on day 1 RM-1 tumours were established in the prostate of C57BL/6 mice by intra prostatic injection of 5 x 10³ RM-1 cells (~ 50 µl cell suspension) per mouse. On day 4, these mice received an intraprostatic injection of 1 x 10¹⁰ VP / tumour of OAdV220, either alone or formulated with 10 µM CS87 (see groups below). Fludarabine was then administered ip at 600 mg/m²/ day to appropriate groups from days 5-10. On day 6, pseudometastases were induced in the lungs by intravenous injection of 2.5 x 10⁵ RM-1 cells (∼ 50 µl cell suspension) per mouse. Due to the limitations of surgery only 30 intraprostate injections can be performed per day. The experiment was therefore set up on 4 different days, although care was taken not to upset the timing of the experiment. The mice were weighed twice weekly, and sacrificed on day 18. Tissues were harvested for analysis (prostate volume, prostate weights).

Treatment groups were as follows:
- Group A: OAdV220 alone ± fludarabine
- Group B: Nil treatment (storage buffer) ± fludarabine
- Group C: OAdV220 plus 10 µM CS87 ± fludarabine
- Group D: 10 µM CS87 alone ± fludarabine

Analysis of tumour volumes in the prostate revealed considerable variation between mice, possibly because the architecture of the organ was disrupted when the intraprostate virus injections were given. Moreover, there were some differences between the results obtained on different days, due to experimental variability. However, within each group (plus or minus Fludarabine in one situation per day) the results were consistent. The results of this study, shown in Figure 10, indicate that Fludarabine alone caused some repression of tumour growth (17% decrease in tumour weight and 12% decrease in tumour volume when compared with tumours receiving no treatment. Similarly OAdV220 plus fludarabine, or CS87 plus fludarabine caused 27% and 30% reduction in tumour volume, and 18% and 23% reduction in tumour weights respectively when compared with the appropriate virus or lipid alone controls. The most marked effect on tumour growth was seen with a combination of OAdV220 plus CS87 plus fludarabine. This combined therapy decreased tumour volume by 58% (p<0.05, *) and tumour weight by 53% (p<0.05, *).

### Example 9. OAdV viruses carrying the PNP gene under the control of the chimaeric PSM/PB promoter displays prostate specific PNP expression.

A chimaeric promoter carrying the 1kb PSM enhancer coupled to the 430 bp proximal promoter from the rat probasin gene (PSM/PB) was linked to the PNP gene/bovine growth hormone polyadenylation sequences. This expression cassette was incorporated into the OAdV genome at two different insertion sites (site I and site III), producing viruses OAdV223 and OAdV623, respectively (see Figure 1). Different cell types were infected with OAdV223, OAdV623 or viruses in which the PNP gene was under the control of the constitutive RSV or CMV promoters (OAdV220 and OAdV222 respectively). Two days after infection with the appropriate virus, cell extracts were prepared and PNP activity was measured as described in example 2 but using 6MPDR as a substrate.

Figure 11 shows that, in the context of the viral genome and OAdV infection, the PSM/PB promoter retained a high degree of tissue specificity. OAdV623 produced a greater level of gene expression than OAdV223 in the human prostate cancer cell types. In LNCaP and LN3 prostate cancer cells expression from the PSM/Pb element produced considerably more (4-8 fold) PNP activity than the RSV promoter but, significantly, in non-prostate cell lines these relative activities were reversed. In these latter cell types expression favoured the RSV promoter, about 4 and 5 fold for MCF-7 and HEK293 cells, 8 fold for HepG2 cells and 32 fold for MRC-5 cells (Figure 11). Using RSV promoter activity to normalise the data, the nett effect is a preferential level of PSM/Pb promoter activity in LN3 and LNCaP cells of 15-40 fold over MCF-7 and HEK293 cells, 32-64 fold over HepG2 cells and 128-256 fold over MRC5 cells, respectively.

### Example 10. An OAdV-delivered, prostate-specific GDEPT is active against human prostate tumours grown in Nude (Immune-suppressed) Mice

OAdV623 contains an expression cassette carrying the PNP gene under the control of the prostate-specific promoter, PSM/Pb. This promoter is only active in human prostate cells. To test whether this virus in combination with lipid and prodrug could elicit an antitumour response against human prostate cancer, tumours derived from the human prostate cancer cell line LN3 were established in nude mice and treated as described below.

LN3 tumours were established in male nude mice by subcutaneous injection of 2 x 10⁶ cells diluted 1:1 with matrigel (100 µl) and allowed to grow to a 5 x 5 mm size. Ten tumour bearing mice per group were injected intratumorally with either vehicle only (Vehicle Control and Fludarabine Control) or OAdV623 at 10¹⁰ VP/tumour plus 10 µM CS87 (GDEPT) on day 0. Animals in the Fludarabine Control and GDEPT groups then received fludarabine phosphate intraperitoneally at 75 mg/ m²/ day on days 1 to 5. Animal weights and tumour volumes were recorded twice weekly. The median relative tumour volume for the GDEPT group was then compared to that of the Vehicle Control and Fludarabine Control groups.

The effects of treatment on tumour growth are shown in Figure 12(upper). Whereas the control groups showed tumour doubling times of 5 to 10 days, the doubling time in the GDEPT group was 17 days. On Day 20 post-treatment, median tumour size in the GDEPT group was 50% smaller than in the Vehicle Control group.

Figure 12 (lower) shows that the combination of OAdV623 with fludarabine treatment in the presence of lipid substantially extended the life of the tumour bearing animals. Median survival was 27 days in the Vehicle Control group and 48 days in the GDEPT group (P<0.02, Logrank Test).

## Claims

1. A composition comprising:
(i) an engineered ovine atadenovirus comprising a probasin promoter, a transcriptional enhancer sequence from the prostate specific membrane antigen (PSMA) gene, and a gene encoding a purine nucleoside phosphorylase (PNP) enzyme, which enzyme converts a purine prodrug to a toxic purine metabolite; and
(ii) a CS87 lipid having the formula: 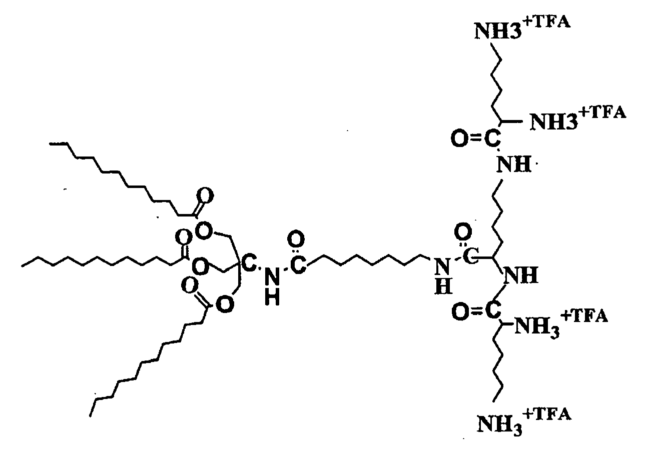 or a CS60 lipid having the formula: 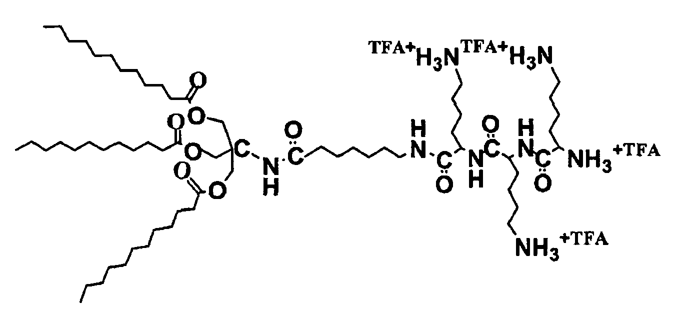 for use in conjunction with a purine prodrug in the treatment of a solid tumour in a subject.

2. The use of a composition comprising:
(i) an engineered ovine atadenovirus comprising a probasin promoter, a transcriptional enhancer sequence from the prostate specific membrane antigen (PSMA) gene, and a gene encoding a purine nucleoside phosphorylase (PNP) enzyme, which enzyme converts a purine prodrug to a toxic purine metabolite; and
(ii) a CS87 lipid having the formula: 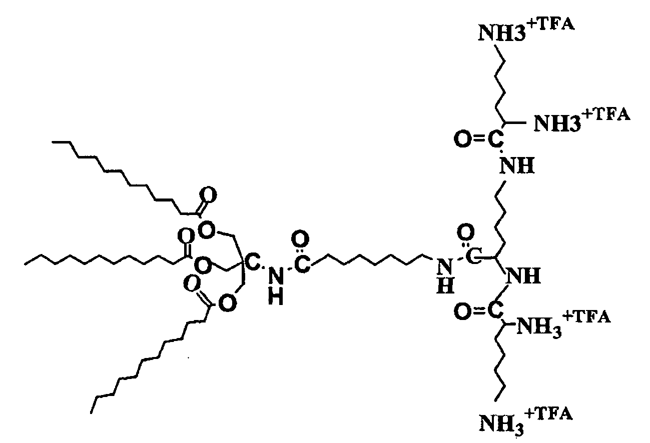 or a CS60 lipid having the formula: 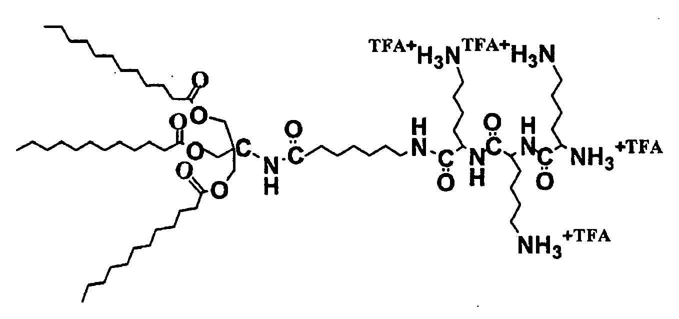 in the manufacture of a medicament for use in conjunction with a purine prodrug, for the treatment of a solid tumour in a subject.

3. The composition of claim 1 or use of claim 2 in which the purine prodrug is 6-methyl purine-2-deoxyriboside (6MPDR) or fludarabine.

4. The composition or use of any preceding claim in which the engineered ovine atadenovirus is selected from the group consisting of OAdV223 and OAdV623.

5. The composition or use of any preceding claim in which the solid tumour to be treated is prostate cancer.

## Patentansprüche

1. Zusammensetzung, die aufweist:
(i) ein biotechnologisch erzeugtes Schaf-Atadenovirus, das einen Probasin-Promotor, eine transkriptionelle Verstärkersequenz aus dem Gen für das Prostataspezifische Membranantigen (PSMA), und ein Gen, das für ein Purin-Nukleosid-Phosphorylase(PNP)-Enzym codiert, aufweist, wobei dieses Enzym eine Purin-Prodrug in einen toxischen Purin-Metaboliten umwandelt; und
(ii) ein CS87-Lipid der Formel: 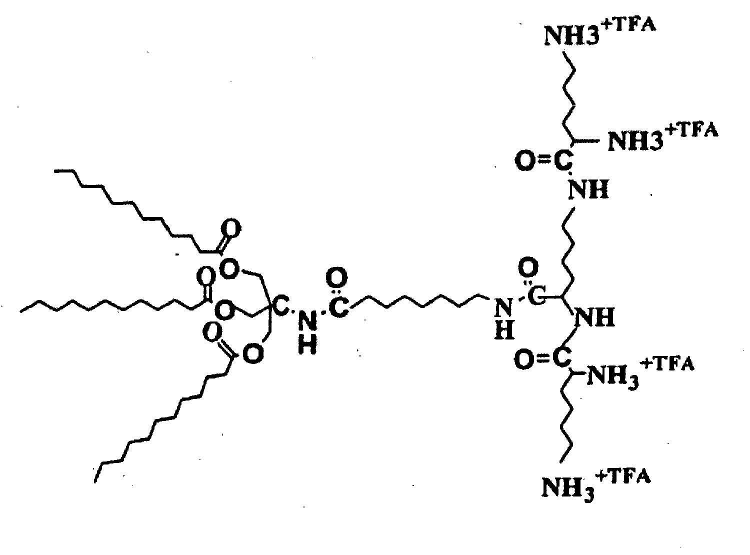 oder ein CS60 Lipid mit der Formel: 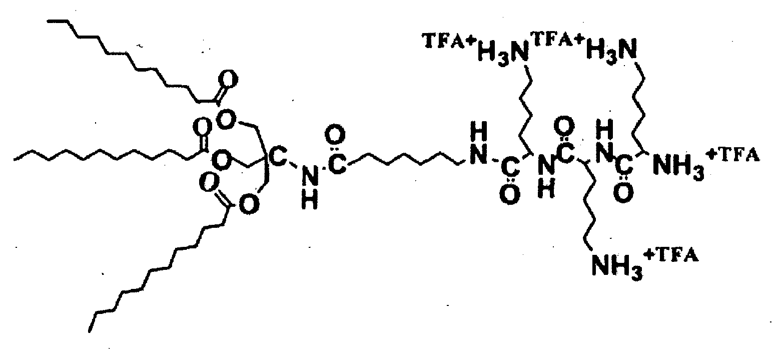 zur Verwendung gemeinsam mit einer Purin-Prodrug bei der Behandlung eines festen Tumors bei einer Person.

2. Verwendung einer Zusammensetzung, die umfasst:
(i) ein biotechnologisch erzeugtes Schaf-Atadenovirus, das einen Probasin-Promotor, eine transkriptionelle Verstärkersequenz aus dem Gen für das Prostataspezifische Membranantigen (PSMA), und ein Gen, das für ein Purin-Nukleosid-Phosphorylase(PNP)-Enzym codiert, aufweist, wobei dieses Enzym eine Purin-Prodrug in einen toxischen Purin-Metaboliten umwandelt; und
(ii) ein CS87-Lipid der Formel: 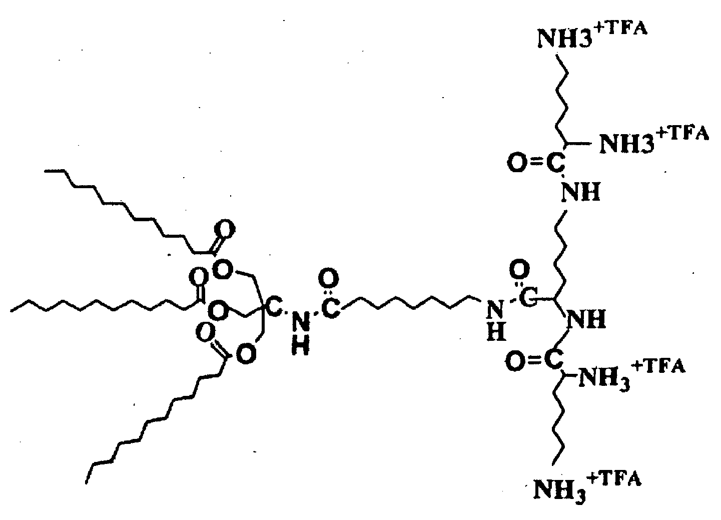 oder ein CS60 Lipid mit der Formel: 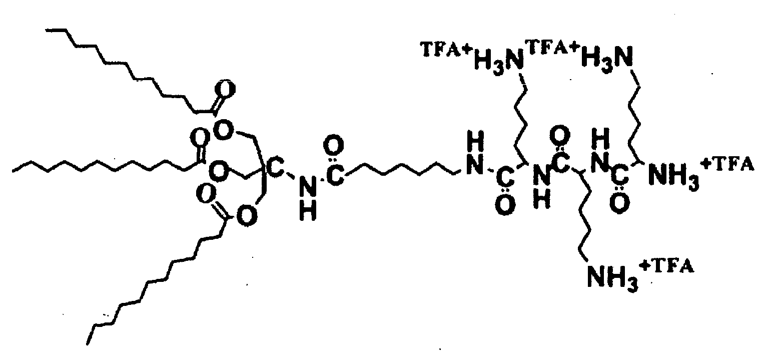 bei der Herstellung eines Medikaments zur Verwendung gemeinsam mit einer Purin-Prodrug zur Behandlung eines festen Tumors bei einer Person.

3. Zusammensetzung nach Anspruch 1 oder Verwendung nach Anspruch 2, wobei die Purin-Prodrug 6-Methylpurin-2-desoxy-ribosid (6MPDR) oder Fludarabin ist.

4. Zusammensetzung oder Verwendung nach irgendeinem vorausgehenden Anspruch, wobei das gentechnisch erzeugte Schaf-Atadenovirus ausgewählt ist aus der Gruppe, die besteht aus OAdV223 und OAdV623.

5. Zusammensetzung oder Verwendung nach irgendeinem vorausgehenden Anspruch, wobei der zu behandelnde feste Tumor Prostatakrebs ist.

## Revendications

1. Composition comprenant :
(i) un atadénovirus ovin modifié comprenant un promoteur de la probasine, une séquence amplificatrice transcriptionnelle provenant du gène de l'antigène prostatique spécifique membranaire (PSMA), et un gène codant pour une enzyme purine nucléoside phosphorylase (PNP), enzyme qui convertit un promédicament à base de purine en un métabolite purinique toxique ; et
(ii) un lipide CS87 possédant la formule : 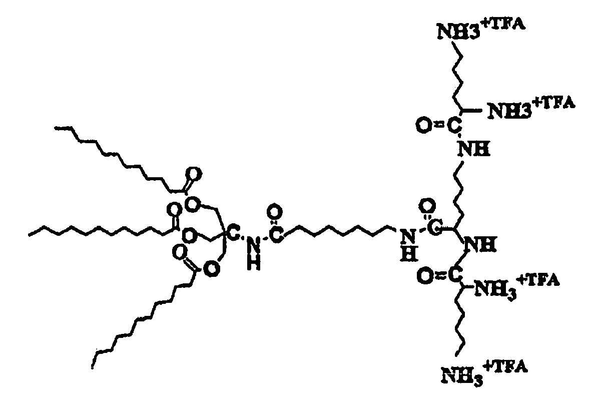 ou un lipide CS60 possédant la formule : 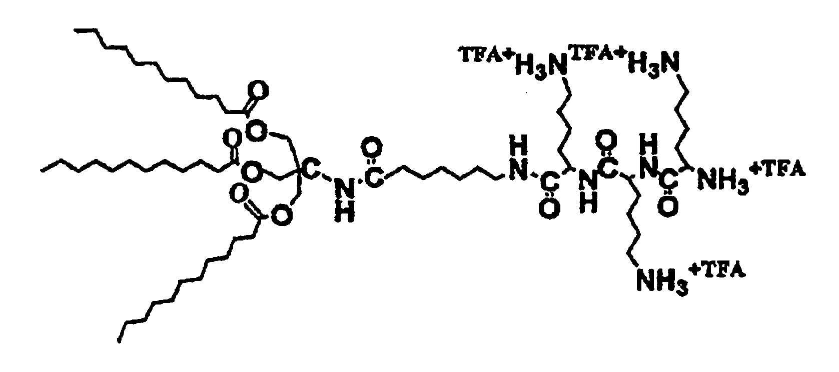 à utiliser conjointement avec un promédicament à base de purine dans le traitement d'une tumeur solide chez un sujet.

2. Utilisation d'une composition comprenant :
(i) un atadénovirus ovin modifié comprenant un promoteur de la probasine, une séquence amplificatrice transcriptionnelle provenant du gène de l'antigène prostatique spécifique membranaire (PSMA), et un gène codant pour une enzyme purine nucléoside phosphorylase (PNP), enzyme qui convertit un promédicament à base de purine en un métabolite purinique toxique ; et
(ii) un lipide CS87 possédant la formule : 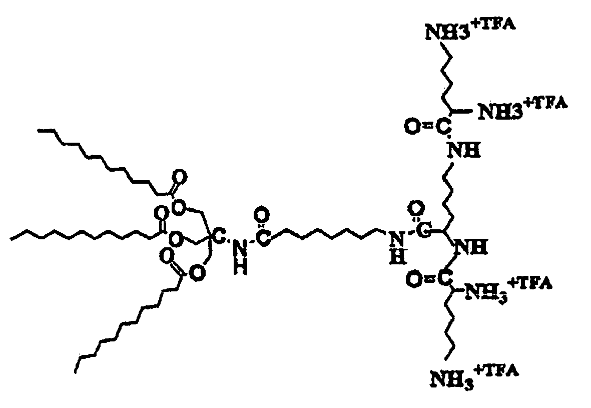 ou un lipide CS60 possédant la formulé : 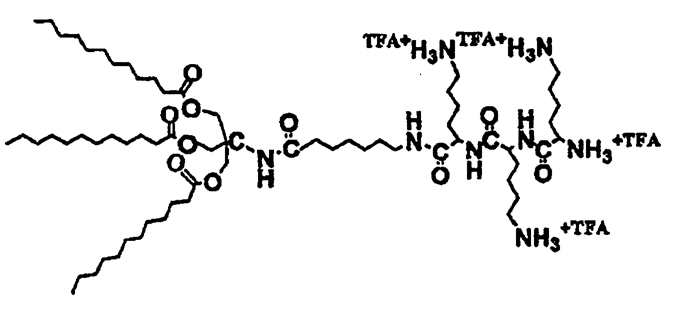 dans la fabrication d'un médicament à utiliser conjointement avec un promédicament à base de purine, pour le traitement d'une tumeur solide chez un sujet.

3. Composition selon la revendication 1 ou utilisation selon la revendication 2, dans laquelle le promédicament à base de purine est le 6-méthylpurine-2-désoxyriboside (6MPDR) ou la fludarabine.

4. Composition ou utilisation selon l'une quelconque des revendications précédentes, dans laquelle l'atadénovirus ovin modifié est choisi dans le groupe constitué de OAdV223 et OAdV623.

5. Composition ou utilisation selon l'une quelconque des revendications précédentes, dans laquelle la tumeur solide à traiter est un cancer de la prostate.
